# EUROPÄISCHE PATENTSCHRIFT

(11) **EP 1 100 748 B1**
(45) Veröffentlichungstag und Bekanntmachung des Hinweises auf die Patenterteilung: **16.04.2003**
(21) Anmeldenummer: 99940153.2
(22) Anmeldetag: 05.08.1999
(51) Int. Cl.: C01B 13/02

(54) **VORRICHTUNG UND VERFAHREN ZUR PRODUKTION VON SINGULETT-SAUERSTOFF**
DEVICE AND METHOD FOR THE PRODUCTION OF SINGLET OXYGEN
DISPOSITIF ET PROCEDE PERMETTANT DE PRODUIRE DE L'OXYGENE SINGULET

(30) Priorität: 05.08.1998 DE 19835456
(43) Veröffentlichungstag der Anmeldung: 23.05.2001
(73) Patentinhaber: Fraunhofer-Gesellschaft zur Förderung der angewandten Forschung e.V., 80636 München (DE)
(72) Erfinder: BRUCKER, Franz, D-79102 Freiburg (DE); KÖHL, Michael, D-79379 Britzingen (DE); LIESKE, Volker, D-79112 Freiburg (DE)
(74) Vertreter: Pfenning, Meinig & Partner
(86) Internationale Anmeldenummer: EP9905667
(87) Internationale Veröffentlichungsnummer: WO00007933

(56) Entgegenhaltungen:
- DE-A- 3 836 759
- DE-U- 29 702 122
- US-A- 4 436 715
- US-A- 4 579 837
- US-A- 4 849 076
- DATABASE WPI Section PQ, Week 199744 Derwent Publications Ltd., London, GB; Class P31, AN 1997-471665 XP002125787 & CN 1 119 518 A (DONG G), 3. April 1996 (1996-04-03)

## Beschreibung

Die Erfindung betrifft eine Vorrichtung und ein Verfahren zur Produktion von Singulett-Sauerstoff aus Triplett-Sauerstoff.

Sauerstoff liegt im Grundzustand als Triplett-Molekül vor und läßt sich durch Energiezufuhr in den angeregten Singulett-Zustand überführen. Angeregter Singulett-Sauerstoff ist besonders reaktionsfreudig und wird beispielsweise in der chemischen Prozeßtechnik, in der Medizin und in der Wasseraufbereitung eingesetzt.

Bisher wird Singulett-Sauerstoff entweder durch chemische Reaktionen oder durch Bestrahlen einer photoempfindlichen Schicht, welche sich in Kontakt zu Luft oder reinem Sauerstoff befindet, erzeugt. Durch Quenchen der Fluoreszenz werden die Triplett-Sauerstoffmoleküle durch einen strahlungslosen Übergang in den angeregten Singulett-Zustand versetzt. Da Singulett-Sauerstoff in Lösung nur eine sehr kurze Lebensdauer aufweist, wird er in der Regel in gasförmigem Zustand zum Einsatzort gebracht.

In der Krebstherapie ist es üblich, den Photosensibilisator durch eine intravenöse Injektion im Körper zu verteilen. Anschließend wird zur lokalen Erzeugung von Singulett-Sauerstoff im Bereich des erkrankten Gewebes endoskopisch Licht eingekoppelt. Bei Hautkrankheiten wird im Anschluß an ein Injizieren des Photosensibilisators die erkrankte Hautstelle zur lokalen Erzeugung von Singulett-Sauerstoff von außen mit Licht bestrahlt. Das Injizieren des Photosensibilisators hat jedoch den Nachteil, daß die Patienten aufgrund von Nebenwirkungen in Folge unkontrollierter Bestrahlung im Anschluß an die Behandlung mindestens vier Wochen in verdunkelten Räumen verweilen müssen, bis der Photosensibilisator abgebaut oder ausgeschieden ist.

Ausgehend von diesem Problem liegt der Erfindung die Aufgabe zugrunde, eine Vorrichtung zum Erzeugen von Singulett-Sauerstoff anzugeben, welche eine besser dosierbare lokale Erzeugung von Singulett-Sauerstoff erlaubt. Die Vorrichtung soll insbesondere eine Singulett-Sauerstoff-Behandlung von Patienten mit weniger Nebenwirkungen gestatten. Aufgabe der Erfindung ist weiterhin, ein Verfahren zum lokalen Erzeugen von Singulett-Sauerstoff anzugeben.

Diese Aufgaben werden gelöst durch eine Vorrichtung gemäß Anspruch 1 und ein Verfahren gemäß Anspruch 13. Die jeweiligen Unteransprüche beinhalten bevorzugte Weiterbildungen und Ausgestaltungen der Erfindung.

Es wird eine Vorrichtung zum Erzeugen von Singulett-Sauerstoff aus Triplett-Sauerstoff vorgeschlagen, die eine Lichtquelle und ein transparentes Medium, welches eine erste und eine zweite Oberfläche aufweist, enthält. Während über die erste Oberfläche an der Rückseite des transparenten Mediums Licht von der Lichtquelle einkoppelbar ist, ist die zweite Oberfläche an der Vorderseite mit einem einen oder mehrere Photosensibilisatoren enthaltenden Bereich versehene. Dieser Bereich ist eine Beschichtung oder ein Träger für den Photosensibilisator, der auf der zweiten Oberfläche angeordnet ist und den Photosensibilisator z.B. in Form einer Beschichtung, oder eingebettet in eine gegebenenfalls poröse Matrix enthält. Der Bereich kann zur Oberflächenvergrößerung auch eine aufgerauhte Oberfläche aufweisen.

Zur Erzeugung von Singulett-Sauerstoff wird das erzeugte Licht zunächst über die erste Oberfläche in das transparente Medium eingekoppelt und innerhalb des transparenten Mediums zu der zweiten Oberfläche geführt. In der Nähe der zweiten Oberfläche tritt das in dem transparenten Medium geführte Licht in Kontakt mit dem Photosensibilisator, welcher sich wiederum in Kontakt zu Triplett-Sauerstoff befindet. Aufgrund einer Wechselwirkung zwischen Photosensibilisator, Triplett-Sauerstoff und Licht bildet sich lokal im Bereich der zweiten Oberfläche des transparenten Mediums Singulett-Sauerstoff.

Die erfindungsgemäße lokale Erzeugung von Singulett-Sauerstoff im Bereich der zweiten Oberfläche des transparenten Mediums ist mit einer Reihe von Vorteilen verbunden. So kann beispielsweise bei der Verwendung der erfindungsgemäßen Vorrichtung im medizinischen Bereich auf eine intravenöse Injektion des Photosensibilisators verzichtet werden. Außer im medizinischen Bereich läßt sich die Erfindung jedoch auch auf vielen weiteren Gebieten, welche die lokale Erzeugung von Singulett-Sauerstoff erfordern, verwenden. So kann beispielsweise an den Einsatz in der chemischen Prozeßtechnik gedacht werden.

Für eine großflächige Erzeugung von Singulett-Sauerstoff kann das transparente Medium beispielsweise als transparenter Körper in Form einer die Lichtquelle zumindest bereichsweise umgebenden Abdeckung ausgestaltet sein. Alternativ kann das transparente Medium ein integraler Bestandteil der Lichtquelle wie beispielsweise der Glaskolben einer Lampe oder eine sonstige mit der Lampe fest verbundene Abdeckung sein. Das transparente Medium wird mit der Oberfläche, auf welcher der Photosensibilisator angeordnet ist, in die Nähe des Einsatzortes gebracht und auf der anderen Oberfläche von der Lichtquelle beleuchtet.

Für die lokale Erzeugung von Singulett-Sauerstoff in schwer zugänglichen Bereichen kann das transparente Medium als Lichtwellenleiter ausgestaltet sein, der auf einer Stirnfläche mit einem den Photosensibilisator enthaltenden Bereich versehen ist, wobei über die andere Stirnfläche das Licht endoskopisch eingekoppelt werden kann.

Weitere Einzelheiten und bevorzugte Ausgestaltungen der Erfindung ergeben sich aus den Figuren und den Ausführungsbeispielen. Es zeigen:
- Fig. 1: eine Vorrichtung zur lokalen großflächigen Erzeugung von Singulett-Sauerstoff;
- Fig. 2: eine Vorrichtung zur lokalen Erzeugung von Singulett-Sauerstoff in schwer zugänglichen Bereichen; und
- Fig. 3: eine Vorrichtung zur lokalen Erzeugung von Singulett-Sauerstoff mit einem abnehmbaren Träger für den Photosensibilisator.

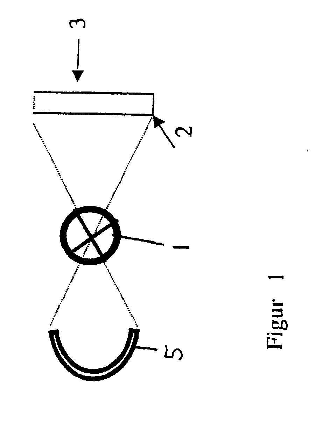

In Fig. 1 ist eine Vorrichtung zur großflächigen lokalen Erzeugung von Singulett-Sauerstoff mit einer Lichtquelle 1, einem planaren transparenten Körper 2 und einer einen Photosensibilisator enthaltenden Schicht 3 dargestellt. Als Lichtquelle 1 kann eine breitbandige Lampe, z.B. in Verbindung mit einem Reflektor 5, ein Laser z.B. in Verbindung mit einem Diffusor, oder auch die Sonne verwendet werden. Der transparente Körper 2, welcher auf der Vorderseite mit einer Photosensibilisatorschicht 3 versehen ist, wird von der Rückseite mit Licht von der Lichtquelle 1 bestrahlt. Die Vorrichtung gemäß Fig. 1 eignet sich z.B. zur Behandlung von Hautkrankheiten.

Anstatt die Schicht 3 direkt auf eine Oberfläche des transparenten Körpers 2 aufzubringen, kann auch daran gedacht werden, die Schicht 3 auf einen z.B. transparenten Träger aufzubringen, welcher lösbar auf der Oberfläche des transparenten Körpers 2 angeordnet ist.

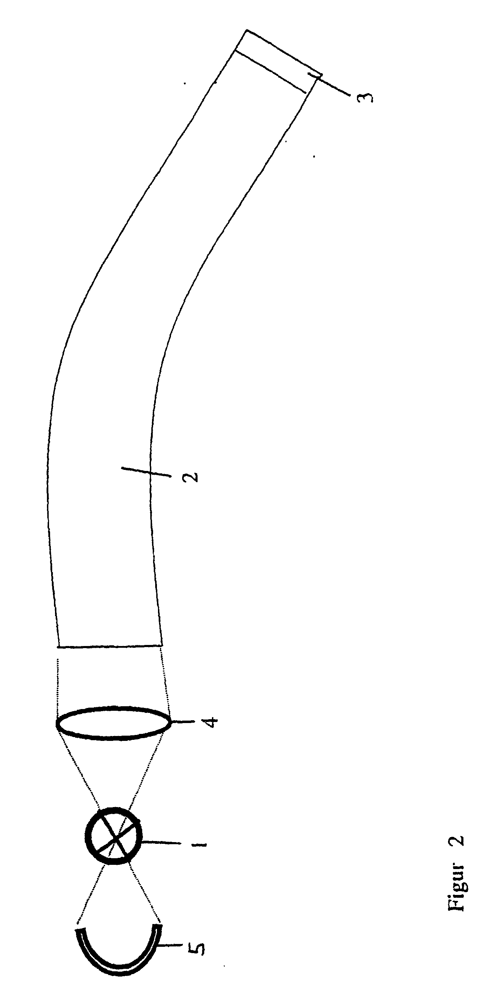

In Fig. 2 ist eine Vorrichtung zur lokalen Erzeugung von Singulett-Sauerstoff in schwer zugänglichen Bereichen, z.B. im Körperinneren, dargestellt. Die Vorrichtung enthält eine Lichtquelle 1 und eine Glasfaser als Lichtwellenleiter 2. Mit Hilfe einer Einkoppeloptik 4, z.B. einer Linse, wird das von der Lichtquelle 1 erzeugte Licht über eine Stirnfläche des Lichtwellenleiters in den Lichtwellenleiter eingekoppelt. Auf der anderen Stirnfläche des Lichtwellenleiters 2 ist eine einen Photosensibilisator enthaltende Schicht 3 oder ein auswechselbarer und einen Photosensibilisator enthaltender Träger aufgebracht. Diese Stirnfläche wird anschließend zur Behandlung zum Einsatzort geführt.

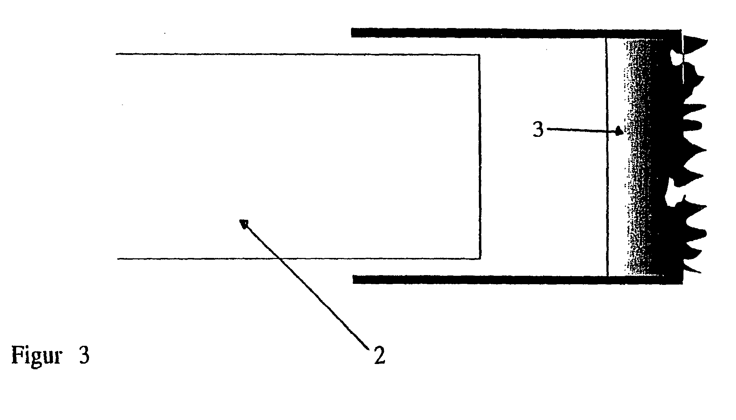

In Fig. 3 ist eine Abwandlung der in Fig. 2 dargestellten Vorrichtung abgebildet. Der den Photosensibilisator enthaltende Bereich ist als Träger 3 ausgestaltet, der auf den Lichtwellenleiter 2 aufgesetzt ist. Der Träger 3 umfaßt ein Befestigungsrohr, welches an einer Stirnfläche mit einem den Photosensibilisator enthaltenden Substrat abgeschlossen ist. Die Substratoberfläche ist aufgerauht, um die aktive Oberfläche für die Singulett-Sauerstoffproduktion zu vergrößern. Grundsätzlich kann der Träger 3 z.B. Glassubstrate, poröse Glasfilter oder transparente Polymere, welche z.B. auf der Außenseite mit dem Photosensibilisator beschichtet sind oder ganzvolumig mit dem Photosensibilisator durchsetzt sind, enthalten. Der Träger kann mechanisch oder adhesiv mit dem transparenten Medium 2 verbunden sein.

Die in den Figuren 1, 2 und 3 dargestellte Schicht 3 bzw. der Träger 3 enthalten als Photosensibilisator z.B. einen Stoff, der ausgewählt ist aus Rose Bengal, Kupfer(II)-Phthalocyanin, 5-Aminolävulinsäure, Porphyrinen, Phthalocyaninen, Chlorinen, Tetraphenylporphyrinen, Benzoporphyrin-Derivaten, Purpurinen, Pheophorbiden und deren Metallkomplexen. Für den Einsatz der Vorrichtungen in sauerstoffarmer Umgebung kann der Bereich 9 zur Speicherung von Sauerstoff porös ausgestaltet sein. So kann beispielsweise ein poröser Photosensibilisator oder aber auch ein poröser Träger für den Photosensibilisator verwendet werden. Auch kann zusammen mit dem Photosensibilisator eine sauerstoffspendende chemische Verbindung wie beispielsweise organische oder anorganische Peroxide auf das transparente Medium 2 aufgebracht werden. Sauerstoff kann auch durch freie Konvektion oder aus Vorratsbehältern der Schicht 3 zugeführt werden. Bei der in der Fig. 2 dargestellten Vorrichtung kann ein mit dem Lichtwellenleiter geführter Schlauch die direkte Zuführung von Sauerstoff zum Photosensibilisator ermöglichen.

Anstatt Sauerstoff kann bei den Vorrichtungen gemäß den Figuren 1 bis 3 auch ein sauerstoffhaltiges Gas wie insbesondere Luft der Schicht 3 zugeführt werden. Es kann auch Photosensibilisator nachgeliefert werden.

## Patentansprüche

1. Vorrichtung zur Produktion von Singulett-Sauerstoff
mit einer Lichtquelle (1) und einem transparenten Medium (2), welches eine erste und eine zweite Oberfläche aufweist, wobei über die erste Oberfläche an der Rückseite Licht der Lichtquelle (1) in das transparente Medium (2) einkoppelbar ist und auf der zweiten Oberfläche an der Vorderseite ein als Beschichtung oder als Träger ausgestalteter Bereich (3) angeordnet ist, der mindestens einen Photosensibilisator enthält.

2. Vorrichtung nach Anspruch 1,
**dadurch gekennzeichnet, daß** eine Vorrichtung zur Zuführung von Sauerstoff und/oder eines sauerstoffhaitigen Gases und/oder Photosensibilisator zum Bereich (3) vorgesehen ist.

3. Vorrichtung nach einem der Ansprüche 1 oder 2, **dadurch gekennzeichnet, daß** die Lichtquelle (1) die Sonne, eine Lampe oder ein Laser ist.

4. Vorrichtung nach einem der Ansprüche 1 bis 3, **dadurch gekennzeichnet, daß** der Photosensibilisator ausgewählt ist aus Porphyrinen, Phthalocyaninen, Chlorinen, Tetraphenylporphyrinen, Benzoporphyrin-Derivaten, Purpurinen, Pheophorbiden und deren Metallkomplexen.

5. Vorrichtung nach einem der Ansprüche 1 bis 4, **dadurch gekennzeichnet, daß** der Photosensibilisator Kupfer(II)-Phthalocyanin, Rose Bengal oder 5-Aminoävulinsäure ist.

6. Vorrichtung nach einem der Ansprüche 1 bis 5, **dadurch gekennzeichnet, daß** der Bereich (3) porös ist und/oder eine vergrößerte aktive Oberfläche aufweist.

7. Vorrichtung nach einem der Ansprüche 1 bis 6, **dadurch gekennzeichnet, daß** der Bereich (3) eine sauerstoffspendende chemische Verbindung, wie z.B. organische oder anorganische Peroxide, enthält.

8. Vorrichtung nach einem der Ansprüche 1 bis 7, **dadurch gekennzeichnet, daß** das transparente Medium (2) integraler Bestandteil der Lichtquelle (1) ist.

9. Vorrichtung nach einem der Ansprüche 1 bis 7, **dadurch gekennzeichnet, daß** das transparente Medium (2) eine die Lichtquelle (1) zumindest bereichsweise umgebende Abdeckung ist.

10. Vorrichtung nach einem der Ansprüche 1 bis 7, **dadurch gekennzeichnet, daß** das transparente Medium (2) ein Lichtwellenleiter ist und die erste Oberfläche von einer ersten Stirnfläche des Lichtwellenleiters und die zweite Oberfläche von einer zweiten Stirnfläche des Lichtwellenleiters gebildet ist.

11. Vorrichtung nach Anspruch 10, **dadurch gekennzeichnet, daß** vor der ersten Oberfläche eine Einkoppel-Optik (4) zum Einkoppeln des Lichtes der Lichtquelle (1) angeordnet ist.

12. Vorrichtung nach einem der Ansprüche 10 oder 11, **dadurch gekennzeichnet, daß** eine zusammen mit dem Lichtwellenleiter führbare Vorrichtung zur Emission von Sauerstoff und/oder eines sauerstoffhaltigen Gases und/oder Photosensibilisator vorgesehen ist.

13. Verfahren zum Erzeugen von Singulett-Sauerstoff aus Triplett-Sauerstoff, enthaltend die Schritte
- Erzeugen von Licht:
- Einkoppeln des Lichtes über eine erste Oberfläche an der Rückseite in ein transparentes Medium (2);
- Führen des Lichtes innerhalb des transparenten Mediums (2) zu einer zweiten Oberfläche an der Vorderseite des transparenten Mediums (2), auf welcher ein mindestens einen Photosensibilisator enthaltender und als Beschichtung oder als Träger ausgestalteter Bereich (3) angeordnet ist, der sich in Kontakt zu Triplett-Sauerstoff befindet;
- photoinduzierte Anregung des Triplett-Sauerstoffs zu Singulett-Sauerstoff mit Hilfe des Photosensibilisators.

14. Verfahren nach Anspruch 13,
**dadurch gekennzeichnet, daß** Sauerstoff und/oder ein sauerstoffhaltiges Gas und/oder Photosensibilisator zum Bereich (3) geführt wird.

15. Verfahren nach Anspruch 13 oder 14,
**dadurch gekennzeichnet, daß** als transparentes Medium (2) eine Abdeckung, ein integraler Bestandteil einer Lichtquelle (1) oder ein Lichtwellenleiter verwendet wird.

16. Verfahren nach einem der Ansprüche 13 bis 15, **dadurch gekennzeichnet, daß** das Licht mit Hilfe einer Einkoppeloptik (4) in das transparente Medium eingekoppelt wird.

17. Verfahren nach einem der Ansprüche 13 bis 16, **dadurch gekennzeichnet, daß** in dem den Photösensibilisator enthaltenden Bereich (3) Sauerstoff und/oder ein sauerstoffhaltiges Gas gespeichert wird.

18. Verfahren nach einem der Ansprüche 13 bis 17, **dadurch gekennzeichnet, daß** der Bereich (3) porös ausgestaltet wird und/oder mit einer vergrößerten aktiven Oberfläche versehen ist.

## Claims

1. A device for the production of singlet oxygen with a light source (1) and a transparent medium (2) which incorporates a first and a second surface, whereby light created by the light source (1) can be coupled with the transparent medium (2) via the first surface on the reverse side, and whereby the second surface on the front side incorporates a coating or an area (3) in the form of a substrate which contains at least one photosensitizer.

2. A device according to Claim 1, **characterised in that** it incorporates a device for the supply of oxygen and/or an oxygen-containing gas and/or a photosensitizer within the area (3).

3. A device according to Claim 1 or 2, **characterised in that** the light source (1) consists of the sun, a lamp, or a laser.

4. A device according to one of the preceding Claims 1 to 3, **characterised in that** the photosensitizer has been chosen to consist of porphyrines, phtalocyanines, chlorines, tetrathenylporphyrines, benzoporphyrine derivatives, purpurines, pheophorbides and their metallic compounds.

5. A device according to one of the preceding Claims 1 to 4, **characterised in that** the photosensitizer consists of copper (II) phthalocyanine, rose bengal or 5-amino evuline acid.

6. A device according to one of the preceding Claims 1 to 5, **characterised in that** the area (3) is porous and/or contains an enlarged active surface.

7. A device according to one of the preceding Claims 1 to 6, **characterised in that** the area (3) contains an oxygen-generating chemical compound, such as for example organic or inorganic peroxides.

8. A device according to one of the preceding Claims 1 to 7, **characterised in that** the transparent medium (2) forms an integral component of the light source (1).

9. A device according to one of the preceding Claims 1 to 7, **characterised in that** the transparent medium (2) consists of a cover that at least partly surrounds a light source (1).

10. A device according to one of the preceding Claims 1 to 7, **characterised in that** the transparent medium (2) consists of a lightwave conductor and **in that** the first surface is formed by a first facing surface of the lightwave conductor and **in that** the second surface is formed by a second facing surface of the lightwave conductor.

11. A device according to Claim 10, **characterised in that** it incorporates a coupling optic (4) for the coupling of the light created by the light source (1) prior to the first surface.

12. A device according to one of the preceding Claims 10 or 11, **characterised in that** it incorporates a device for the emission of oxygen and/or an oxygen-containing gas and/or a photosensitizer which can be controlled together with the lightwave conductor.

13. A method for the generating of singlet oxygen from triplet oxygen, which further incorporates the following steps
- the generating of light;
- the coupling of the light with a transparent medium (2) via the first surface on the reverse side;
- the routing of the light within the transparent medium (2) towards a second surface on the front side of the transparent medium (2), on which an area (3) is incorporated which contains at least one photosensitizer and which may take the form of a coating or a substrate, and which is in contact with triplet oxygen;
- the photoinduced conversion of the triplet oxygen into singlet oxygen with the aid of the photosensitizer.

14. A method according to Claim 13, **characterised in that** oxygen and/or an oxygen-containing gas and/or a photosensitizer are supplied for the area (3).

15. A method according to Claim 13 or 14, **characterised in that** a cover, an integral component of a light source (1), or a lightwave conductor are used as a transparent medium (2).

16. A method according to one of the preceding Claims 13 to 15, **characterised in that** the light is coupled into the transparent medium with the aid of a coupling optic (4).

17. A method according to one of the preceding Claims 13 to 16, **characterised in that** oxygen and/or an oxygen-containing gas is stored within the area (3) that contains the photosensitizer.

18. A method according to one of the preceding Claims 13 to 17, **characterised in that** the area (3) is porous and/or contains an enlarged active surface.

## Revendications

1. Dispositif de production d'oxygène singulet avec une source de lumière (1) et un milieu transparent (2), qui présente une première et une deuxième surfaces, où la lumière de la source de lumière (1) peut être introduite dans le milieu transparent (2) par l'intermédiaire de la première surface sur la face arrière et une zone (3), réalisée sous forme de couverture ou de support, est disposée sur la deuxième surface, sur la face avant, laquelle contient au moins un photosensibilisateur.

2. Dispositif selon la revendication 1, **caractérisé en ce qu'**un dispositif pour l'alimentation en oxygène et/ou en un gaz contenant de l'oxygène et/ou en le photosensibilisateur dans la zone (3) est prévu.

3. Dispositif selon l'une quelconque des revendications 1 ou 2, **caractérisé en ce que** la source de lumière (1) est le soleil, une lampe ou un laser.

4. Dispositif selon l'une quelconque des revendications 1 à 3, **caractérisé en ce que** le photosensibilisateur est choisi parmi les porphyrines, les phtalocyanines, les chlorines, les tétraphénylporphyrines, les dérivés de la benzoporphyrine, les purpurines, les phéophorbides et leurs complexes métalliques.

5. Dispositif selon l'une quelconque des revendications 1 à 4, **caractérisé en ce que** le photosensibilisateur est la phtalocyanine de cuivre (II), le rose bengale ou l'acide 5-aminolévulique.

6. Dispositif selon l'une quelconque des revendications 1 à 5, **caractérisé en ce que** la zone (3) est poreuse et/ou présente une surface active agrandie.

7. Dispositif selon l'une quelconque des revendications 1 à 6, **caractérisé en ce que** la zone (3) contient un composé chimique générateur d'oxygène, comme par exemple un peroxyde organique ou inorganique.

8. Dispositif selon l'une quelconque des revendications 1 à 7, **caractérisé en ce que** le milieu transparent (2) est un constituant intégré de la source de lumière (1).

9. Dispositif selon l'une quelconque des revendications 1 à 7, **caractérisé en ce que** le milieu transparent (2) est une couverture entourant au moins partiellement la source lumineuse (1).

10. Dispositif selon l'une quelconque des revendications 1 à 7, **caractérisé en ce que** le milieu transparent (2) est une fibre optique et la première surface est formée d'une première surface frontale de la fibre optique et la deuxième surface est formée d'une deuxième surface frontale de la fibre optique.

11. Dispositif selon la revendication 10, **caractérisé en ce que** devant la première surface, une optique de couplage (4) est disposée pour introduire la lumière de la source lumineuse (1).

12. Dispositif selon l'une quelconque des revendications 10 ou 11, **caractérisé en ce qu'**un dispositif guidable avec la fibre optique est prévu pour l'émission d'oxygène et/ou d'un gaz contenant de l'oxygène et/ou du photosensibilisateur.

13. Procédé de production d'oxygène singulet à partir d'oxygène triplet, contenant les étapes consistant à :
- produire de la lumière ;
- introduire la lumière par une première surface sur la face arrière, dans un milieu transparent (2) ;
- conduire la lumière au sein du milieu transparent (2), vers une deuxième surface sur la face avant du milieu transparent (2), sur laquelle est disposée une zone (3) contenant au moins un photosensibilisateur et réalisée sous forme de couverture ou de support, qui est en contact avec l'oxygène triplet ;
- exciter par photoinduction l'oxygène triplet en oxygène singulet à l'aide du photosensibilisateur.

14. Procédé selon la revendication 13, **caractérisé en ce que** l'oxygène et/ou un gaz contenant de l'oxygène et/ou le photosensibilisateur est conduit dans la zone (3).

15. Procédé selon la revendication 13 ou 14, **caractérisé en ce que** l'on utilise comme milieu transparent (2), une couverture, un constituant intégré à une source lumineuse (1) ou une fibre optique.

16. Procédé selon l'une quelconque des revendications 13 à 15, **caractérisé en ce que** la lumière est introduite à l'aide d'une optique de couplage (4) dans le milieu transparent.

17. Procédé selon l'une quelconque des revendications 13 à 16, **caractérisé en ce que** dans la zone (3) contenant le photosensibilisateur, on accumule de l'oxygène et/ou un gaz contenant de l'oxygène.

18. Procédé selon l'une quelconque des revendications 13 à 17, **caractérisé en ce que** la zone (3) est réalisée sous forme poreuse et/ou est munie d'une surface active agrandie.
